(19) Europäisches Patentamt
European Patent Office
Office européen des brevets

(11) Veröffentlichungsnummer: **0 667 342 A1**

# (12) EUROPÄISCHE PATENTANMELDUNG

(21) Anmeldenummer: **95100310.2**

(22) Anmeldetag: **11.01.95**

(51) Int. Cl.[6]: **C07D 235/14**, A61K 31/17, C07D 215/12, C07D 209/10

(30) Priorität: **24.01.94 DE 4401893**

(43) Veröffentlichungstag der Anmeldung: **16.08.95 Patentblatt 95/33**

(84) Benannte Vertragsstaaten: **AT BE CH DE DK ES FR GB GR IE IT LI LU MC NL PT SE**

(71) Anmelder: **BAYER AG**
**D-51368 Leverkusen (DE)**

(72) Erfinder: **Müller-Gliemann, Dr. Matthias**
**Laibacher Strasse 10**
**D-42697 Solingen (DE)**
Erfinder: **Müller, Dr. Ulrich**
**Neuer Triebel 91**
**D-42111 Wuppertal (DE)**
Erfinder: **Beuck, Dr. Martin, c/o Miles Inc.**
**400 Morgan Lane**
**06516 West Haven CT (US)**
Erfinder: **Wohlfeil, Stefan**
**Tucherweg 25**
**D-40724 Hilden (DE)**

## (54) Substituierte Arylharnstoffe zur Behandlung von Arteriosklerose und Restenose.

(57) Substituierte Arylharnstoffe können hergestellt werden durch Umsetzung entsprechend substituierter Amine mit substituierten Arylisocyanaten. Die substituierten Arylharnstoffe eignen sich als Wirkstoffe in Arzneimitteln, insbesondere zur Inhibition der Proliferation von glatten Muskelzellen und zur Behandlung der Arterioskloose und Restenose.

EP 0 667 342 A1

Die Erfindung betrifft substituierte Arylharnstoffe, Verfahren zu ihrer Herstellung sowie ihre Verwendung in Arzneimitteln, insbesondere zur Behandlung der Arteriosklerose und Restenose.

Die vorliegende Erfindung betrifft substituierte Arylharnstoffe der allgemeinen Formel (I)

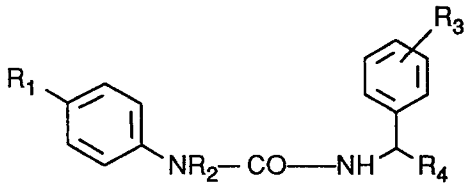

(I)

in welcher

$R^1$ für einen Rest der Formel $-CH_2-A$ oder $-O-CH_2-D$ steht, worin

A und D gleich oder verschieden sind und Wasserstoff oder einen 5- bis 7-gliedrigen, gesättigten oder ungesättigten Heterocyclus mit bis zu 3 Stickstoffatomen bedeuten, an den gegebenenfalls ein Phenyl- oder Pyridylring ankondensiert ist, und wobei die Bindung an die Methylengruppe im Fall eines N-haltigen Ringes auch über die Stickstofffunktion erfolgen kann, und wobei gegebenenfalls die Cyclen bis zu 3-fach gleich oder verschieden durch geradkettiges oder verzweigtes Alkyl, Alkoxy oder Alkoxycarbonyl mit jeweils bis zu 8 Kohlenstoffatomen, Cycloalkyl mit 3 bis 6 Kohlenstoffatomen, Phenyl Hydroxy, Carboxy oder Halogen substituiert sind,

$R^2$ für Cycloalkyl mit 3 bis 7 Kohlenstoffatomen oder für geradkettiges oder verzweigtes Alkyl mit bis zu 8 Kohlenstoffatomen steht,

$R^3$ für Wasserstoff oder Halogen steht,

und

$R^4$ für Carboxy oder geradkettiges oder verzweigtes Alkoxycarbonyl mit bis zu 8 Kohlenstoffatomen steht, oder

für geradkettiges oder verzweigtes Alkyl mit bis zu 6 Kohlenstoffatomen steht, das durch Hydroxy substituiert ist, oder

für eine Gruppe der Formel $-CO-NR^5R^6$ steht,

worin

$R^5$ und $R^6$ gleich oder verschieden sind und Wasserstoff oder geradkettiges oder verzweigtes Alkyl mit bis zu 6 Kohlenstoffatomen bedeuten,

und deren Salze.

Die erfindungsgemäßen substituierten Arylharnstoffe können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Heterocyclus steht im allgemeinen für einen 5- bis 7-gliedrigen, bevorzugt 5- bis 6-gliedrigen, gesättigten oder ungesättigten Ring, der als Heteroatome bis zu 3 Stickstoffatome enthalten kann und an den gegebenenfalls auch ein Phenyl- oder Pyridylring ankondensiert sein kann. Bevorzugt sind benzokondensierte 5-gliedrige Heterocyclen und Chinoline. Beispielsweise seien genannt: Pyrrolyl, Pyrazolyl, Pyridyl,

Pyrimidyl, Pyrazinyl, Pyridazinyl, Imidazolyl, Pyrrolidinyl, Piperidinyl, Piperazinyl, Benzimidazolyl, 3H-Imidazopyridyl, 1-Indolyl, 3-Indolyl oder 2-Chinolinyl. Besonders bevorzugt sind Benzimidazolyl, 3H-Imidazopyridyl, 3-Indolyl oder 2-Chinolinyl.

Bevorzugt sind Verbindungen der allgemeinen Formel (I),

in welcher

$R^1$ für einen Rest der Formel $-CH_2-A$ oder $-O-CH_2-D$ steht,
worin

A und D gleich oder verschieden sind und Wasserstoff, Benzimidazolyl, 3H-Imidazopyridyl, 3-Indolyl oder 2-Chinolinyl bedeuten, die sowohl im Phenyl als auch im heterocyclischen Rest gegebenenfalls bis zu 2-fach gleich oder verschieden durch geradkettiges oder verzweigtes Alkyl, Alkoxy oder Alkoxycarbonyl mit jeweils bis zu 6 Kohlenstoffatomen, Cyclopropyl, Cyclopentyl, Cyclohexyl, Hydroxy, Phenyl, Carboxy, Fluor, Chlor oder Brom substituiert sind,

$R^2$ für Cyclopropyl, Cyclopentyl, Cyclohexyl, Cycloheptyl, oder
für geradkettiges oder verzweigtes Alkyl mit bis zu 6 Kohlenstoffatomen steht,

$R^3$ für Wasserstoff, Fluor, Chlor oder Brom steht,

und

$R^4$ für Carboxy oder geradkettiges oder verzweigtes Alkoxycarbonyl mit bis zu 6 Kohlenstoffatomen oder
für geradkettiges oder verzweigtes Alkyl mit bis zu 4 Kohlenstoffatomen steht, das durch Hydroxy substituiert ist, oder
für eine Gruppe der Formel $-CO-NR^5R^6$ steht,
worin

$R^5$ und $R^6$ gleich oder verschieden sind und Wasserstoff oder geradkettiges oder verzweigtes Alkyl mit bis zu 4 Kohlenstoffatomen bedeuten

und deren Salze.

Besonders bevorzugt sind Verbindungen der allgemeinen Formel (I),

in welcher

$R^1$ für einen Rest der Formel $-CH_2-A$ oder $-O-CH_2-D$ steht,
worin

A und D gleich oder verschieden sind und Wasserstoff, Benzimidazolyl, 3H-Imidazopyridyl, 3-Indolyl oder 2-Chinolinyl bedeuten, die sowohl im Phenyl als auch im heterocyclischen Rest gegebenenfalls bis zu 2-fach gleich oder verschieden durch geradkettiges oder verzweigtes Alkyl, Alkoxy oder Alkoxycarbonyl mit jeweils bis zu 5 Kohlenstoffatomen, Cyclopropyl, Cyclopentyl, Cyclohexyl, Hydroxy, Phenyl oder Carboxy substituiert sind,

$R^2$ für Cyclopropyl, Cyclopentyl, Cyclohexyl, Cycloheptyl, oder
für geradkettiges oder verzweigtes Alkyl mit bis zu 4 Kohlenstoffatomen steht,

$R^3$ für Wasserstoff, Fluor, Chlor oder Brom steht,

und

$R^4$ für Carboxy oder geradkettiges oder verzweigtes Alkoxycarbonyl mit bis zu 3 Kohlenstoffatomen oder
für geradkettiges oder verzweigtes Alkyl mit bis zu 3 Kohlenstoffatomen steht, das durch Hydroxy substituiert ist, oder
für eine Gruppe der Formel $-CO-NR^5R^6$ steht,
worin

$R^5$ und $R^6$ gleich oder verschieden sind und Wasserstoff oder geradkettiges oder verzweigtes Alkyl mit bis zu 3 Kohlenstoffatomen bedeuten

und deren Salze.

Ganz besonders bevorzugt sind Verbindungen der allgemeinen Formel (I),

in welcher

$R^1$ für Wasserstoff oder für den Rest der Formel

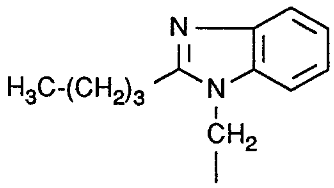
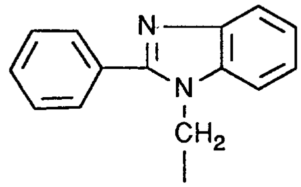
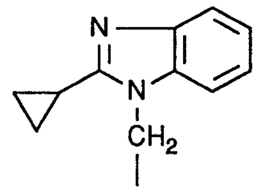

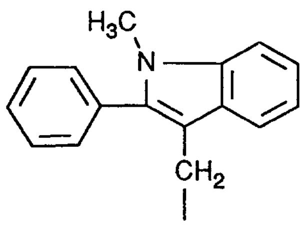

oder

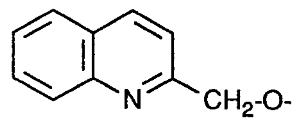

steht.

Außerdem wurde ein Verfahren zur Herstellung der erfindungsgemäßen Verbindungen der allgemeinen Formel (I) gefunden, dadurch gekennzeichnet, daß man Verbindungen der allgemeinen Formel (II)

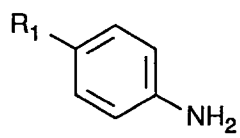

(II)

in welcher
$R^1$ die oben angegebene Bedeutung hat,
zunächst durch Umsetzung mit Verbindungen der allgemeinen Formel (III)

$R^2$-L (III)

in welcher
$R^2$ die oben angegebene Bedeutung hat
und
L für Halogen, vorzugsweise für Brom steht,
in inerten Lösemitteln, in Anwesenheit einer Base und unter Schutzgasatmosphäre in die Verbindungen der allgemeinen Formel (IV)

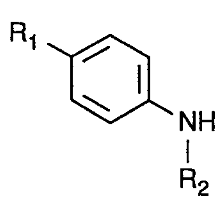

(IV)

in welcher
$R^1$ und $R^2$ die oben angegebene Bedeutung haben,
überführt,
und in einem zweiten Schritt mit Verbindungen der allgemeinen Formel (V)

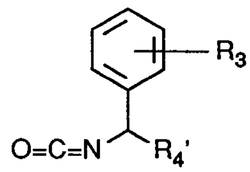

(V)

in welcher
$R^3$ die oben angegebene Bedeutung hat
und

$R^{4'}$ für geradkettiges oder verzweigtes Alkoxycarbonyl mit bis zu 8 Kohlenstoffatomen steht,

in inerten Lösemitteln, gegebenenfalls in Anwesenheit einer Base und unter Schutzgasatmosphäre umsetzt, und im Fall der Säuren $R^4$ = $CO_2H$, die Ester verseift, im Fall der Alkylhydroxyverbindungen die Ester nach üblichen Bedingungen reduziert, und im Fall der Amide die Ester oder Säuren entweder mit Ammoniak ($R^4$ = $CO\text{-}NH_2$) oder mit Aminen, gegebenenfalls nach vorgeschalteter Aktivierung der Carbonsäurefunktion, umsetzt.

Das erfindungsgemäße Verfahren kann durch folgendes Formelschema beispielhaft erläutert werden:

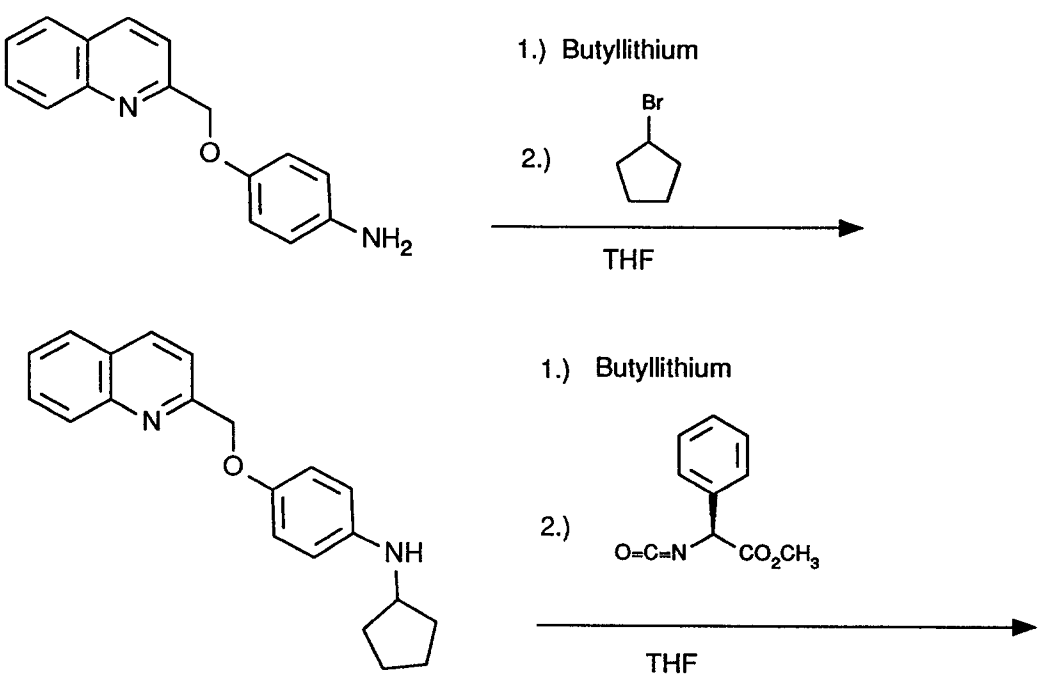

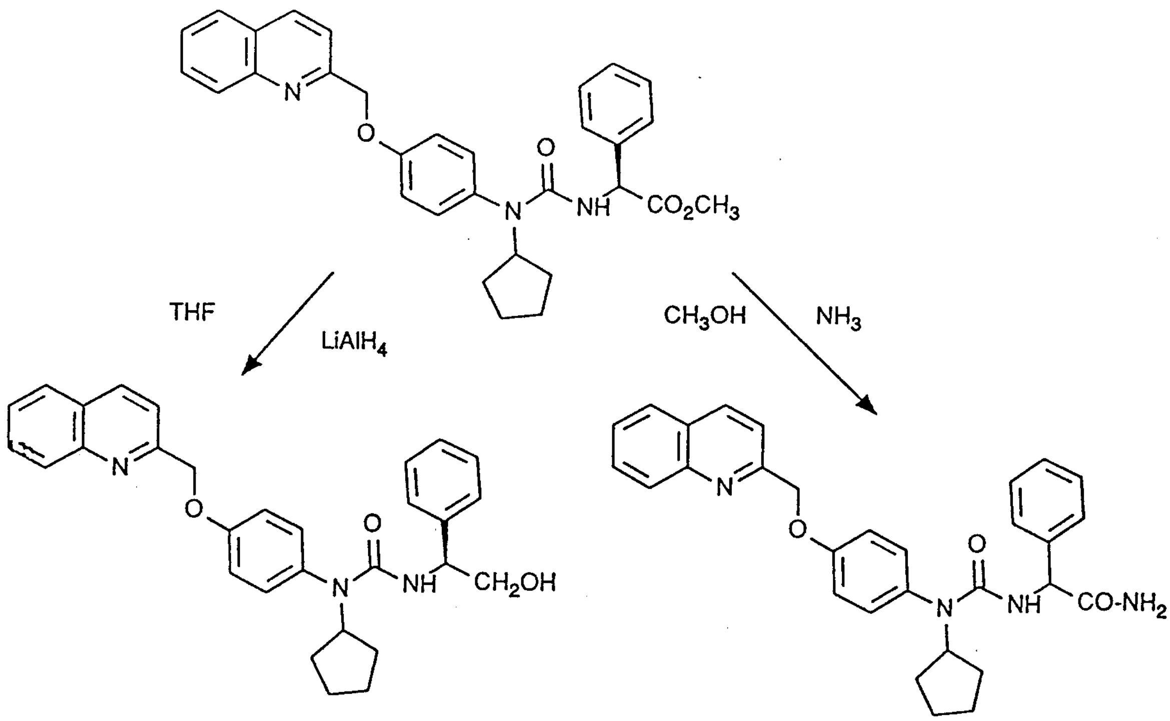


Für die Umsetzungen mit den Verbindungen der allgemeinen Formeln (II) und (IV) eignen sich im allgemeinen inerte Lösemittel, die sich unter den Reaktionsbedingungen nicht verändern. Hierzu gehören vorzugsweise Ether wie beispielsweise Dioxan, Tetrahydrofuran oder Diethylether, Kohlenwasserstoffe wie Benzol, Xylol, Toluol, Hexan, Cyclohexan oder Erdölfraktionen, Dimethylformamid oder Acetonitril. Ebenso ist es möglich, Gemische der Lösemittel einzusetzen. Bevorzugt ist Tetrahydrofuran.

Als Basen für diese Umsetzungen eignen sich Alkali- oder Erdalkalihydroxide wie beispielsweise Natriumhydroxid, Kaliumhydroxid, Lithiumhydroxid oder Bariumhydroxid, Alkalicarbonate wie Natriumcarbonat oder Kaliumcarbonat, Erdalkalicarbonate wie Calciumcarbonat oder Alkali- oder Erdalkalialkoholate oder -amide wie Natrium- oder Kaliummethanolat, Natrium- oder Kaliumethanolat oder Kalium-tert, butylat oder Lithiumdiisopropylamid (LDA), oder n-Butyllithium, oder organische Amine (Trialkyl($C_1$-$C_6$)amine) wie Triethylamin, oder Heterocyclen wie 1,4-Diazabicyclo[2.2.2]octan (DABCO), 1 ,8-Diazabicyclo[5.4.0]undec-7-en (DBU),Pyridin, N,N-Dimethylaminopyridin (DMAP), Methylpiperidin oder Morpholin. Es ist auch möglich, als Basen Alkalimetalle, wie Natrium oder deren Hydride wie Natriumhydrid, einzusetzen. Bevorzugt sind Triethylamin, Lithiumhydroxid, DBU, N,N-Dimethylaminopyridin und n-Butyllithium.

Im allgemeinen setzt man die Base in einer Menge von 0,05 mol bis 10 mol, bevorzugt von 1 mol bis 2 mol bezogen auf 1 mol der Verbindung der Formel (II) ein.

Das erfindungsgemaße Verfahren wird im allgemeinen in einem Temperaturbereich von -30°C bis +100°C, bevorzugt von -10°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Verseifung die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt wird Lithiumhydroxid, Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Dioxan-Wasser-Gemische eingesetzt.

Die Hydrolyse kann auch mit Säuren wie beispielsweise Trifluoressigsäure (TFA), Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden.

Die Amidierung der endständigen Ester ($R^4$ = $-CO-NR^5R^6$) erfolgt im allgemeinen in einem der oben aufgeführten Lösemitteln, vorzugsweise in Alkoholen wie Methanol, Ethanol, Propanol oder Isopropanol, oder in Ethern wie Tetrahydrofuran oder Dioxan, oder in Gemischen derselben, gegebenenfalls unter Wasserzusatz.

Die Amidierung der endständigen Ester kann gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung der endständigen Ester erfolgt im allgemeinen in einem Temperaturbereich von -20 °C bis +80 °C, vorzugsweise von -10 °C bis +30 °C und Normaldruck.

Die Base wird in einer Menge von 0,5 mol bis 10 mol, bevorzugt von 1 mol bis 5 mol, bezogen auf 1 mol der entsprechenden Carbonsäuren bzw. Ester eingesetzt.

Als säurebindende Mittel für die Amidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[4.3.0]-nonene-5 (DBN), 1,5-Diazabicyclo[5.4.0]undecene-5 (DBU) Dimethylaminopyridin, DABCO oder N,N-Dimethylaminopyridin eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexa fluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid, N-Hydroxybenzotriazol oder N-Hydroxysuccinimid. Bevorzugt ist N,N-Dicyclohexylcarbodiimid, gegebenenfalls in Anwesenheit von Triethylamin und N-Hydroxybenzotriazol.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren eingesetzt.

Die Reduktion von Alkoxycarbonylverbindungen zu den entsprechenden Alkoholen erfolgt im allgemeinen mit Hydriden, bevorzugt mit Lithiumaluminiumhydrid in inerten Lösemitteln wie Ethern, Kohlenwasserstoffen oder Alkoholen oder deren Gemischen, bevorzugt in Ethern wie beispielsweise Diethylether, Tetrahydrofuran oder Dioxan, in einem Temperaturbereich von -20 °C bis +150 °C, bevorzugt von 0 °C bis +40 °C, bei Normaldruck.

Die einzelnen Derivatisierungsschritte werden im allgemeinen in einem Temperaturbereich von -78 °C bis +80 °C, bevorzugt von -40 °C bis Raumtemperatur durchgeführt. Gegebenenfalls ist es erforderlich, unter Schutzgasatmosphäre zu arbeiten.

Die Umsetzung der Verbindungen der allgemeinen Formel (IV) erfolgt in einem der oben aufgeführten Lösemittel, wie beispielsweise Tetrahydrofuran, Dioxan, Dimethylformamid oder Toluol. Besonders bevorzugt ist Tetrahydrofuran.

Als Base eignet sich für diesen Schritt eine der oben aufgeführten Basen, vorzugsweise Butyllithium, Kaliumcarbonat oder Triethylamin.

Im allgemeinen setzt man die Base in einer Menge von 0,05 mol bis 10 mol, bevorzugt von 1 mol bis 2 mol jeweils bezogen auf 1 mol der Verbindung der Formel (IV) ein.

Das erfindungsgemäße Verfahren wird im allgemeinen in einem Temperaturbereich von -78°C bis +100°C, bevorzugt von -20°C bis +80°C, durchgeführt.

Die Verbindungen der allgemeinen Formel (II) sind teilweise bekannt oder neu und können beispielweise hergestellt werden, indem man entweder, wie im Fall der methylenoxy-verbrückten Produkte, die entsprechenden Halogenmethylverbindungen mit p-Nitrophenol, oder, wie im Fall der methylen-verbrückten Produkte, die entsprechenden Heterocyclen mit p-Brommethylnitrobenzol in einem der oben aufgeführten Lösemittel, vorzugsweise Dimethylformamid oder Ethanol, in einem Temperaturbereich von -78°C bis +150°C, vorzugsweise bei -20°C bis +100°C umsetzt,
und in einem zweiten Schritt, ebenfalls in einem der oben aufgeführten Lösemittel, vorzugsweise Tetrahydrofuran, nach gängigen Reduktionsmethoden, wie z.B. durch katalytische Hydrierung oder durch Einsatz geeigneter Reduktionsmittel, vorzugsweise durch Verwendung von Titantrichloridlösung unter einem Druck von 0,1 bar reduziert.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I) inhibieren die Proliferation von glatten Muskelzellen. Sie können deshalb zur Behandlung der Arteriosklerose und der Restenose eingesetzt werden.

<u>Untersuchung zur Inhibition der Proliferation glatter Muskelzellen durch die erfindungsgemäßen Verbindungen</u>

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. <u>50</u>, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 $\mu$Ci $^3$H-Thymidin zugefügt und nach weiteren 4 Stunden der Einbau dieser Substanz in die TCA-präzipitierbare DNA der Zellen bestimmt. Zur Bestimmung der $IC_{50}$-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 20 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 50 mg/kg, vorzugsweise 1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

| Laufmittelgemische: | | |
|---|---|---|
| A | Petrolether/Essigester | 1:1 |
| B | Petrolether/Essigester | 1:1,5 |
| C | Toluol/Essigester | 1:3 |
| D | Essigester | |
| E | Petrolether/Essigester | 3:7 |
| F | Toluol/Essigester | 1:1 |
| G | Petrolether/Essigester | 1:3 |

Ausgangsverbindungen

Beispiel I

2-n-Butylbenzimidazol

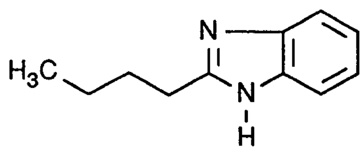

Ein Gemisch aus 10,8 g (0,1 mol) o-Diaminobenzol, 10,9 ml (0,1 mol) Valeriansäure und 100 ml Polyphosphorsäure wird 7 h bei 120 °C gerührt. Anschließend wird auf 1,2 l Eiswasser gegeben, mit NaOH (fest) auf pH 5 eingestellt, mit $Na_2CO_3$ bis zum Ende der Gasentwicklung versetzt und dreimal mit Essigester extrahiert. Die vereinigten organischen Phasen werden über $Na_2SO_4$ getrocknet, filtriert und eingeengt.
Ausbeute: 15,8 g (91 mmol) 91% d.Th.
$R_f$: 0,77 (Essigester/Methanol = 10:1)

Beispiel II

p-[2-(n-Butyl-1H-benzimidazolyl)methyl]nitrobenzol

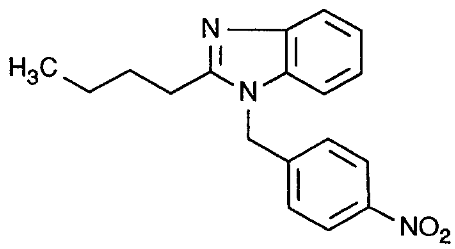

Zu einer Suspension von 6,0 g (0,2 mol) NaH (80%) in 200 ml DMF wird unter Argon bei 0 °C eine Lösung von 34,9 g (0,2 mol) 2-n-Butylbenzimidazol in 250 ml DMF langsam zugetropft. Nach 10 min Rühren bei 0 °C wird eine Lösung von 47,5 g (0,22 mol) p-Nitrobenzylbromid in 250 ml DMF zugetropft, noch 2 h bei 0 °C und 1 h bei Raumtemperatur gerührt. Anschließend wird bei 40 °C eingeengt, der Rückstand mit Ether und Wasser versetzt, geschüttelt, vom Ungelösten abfiltriert, die organische Phase abgetrennt, noch 2 mal mit Ether extrahiert, und die vereinigten organischen Phasen werden über $Na_2SO_4$ getrocknet, filtriert und eingeengt.
Rohausbeute: 78 g
$R_f$(Produkt): 0,47 (Toluol/Essigester = 1:1)
Das Produkt wird ohne Aufreinigung weiter umgesetzt.

Beispiel III

p-[3-(2-Phenyl-1H-indolyl)methyl]nitrobenzol

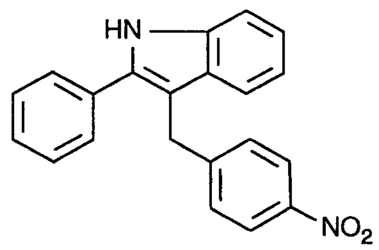

Zu einer Suspension von 0,8 g (30 mmol) NaH (80%) in 30 ml DMF wird bei -10 °C eine Lösung von 5,8 g (30 mmol) 2-Phenylindol in 40 ml DMF getropft und 15 min gerührt. Nach Zutropfen einer Lösung von 7,1 g (33 mmol) p-Nitrobenzylbromid in 40 ml DMF wird 1 h bei -10 °C und 4 h bei Raumtemperatur gerührt. Anschließend wird mit 2 ml Eisessig/10 ml $H_2O$ versetzt und bei 40 °C eingeengt. Der Rückstand wird mit Ether/Wasser verrührt, vom Ungelösten abfiltriert, das Gemisch geschüttelt und getrennt. Die wäßrige Phase wird noch 2 mal mit Ether extrahiert, und die vereinigten organischen Phasen werden über $Na_2SO_4$ getrocknet, filtriert, eingeengt und über Kieselgel 60 chromatographiert (Petrolether / Ether = 7:3).
Ausbeute: 1,1 g (3,3 mmol) 11% d.Th.
$R_f$: 0,41 (Petrolether / Ether = 7:3)

Beispiel IV

p-[3-(2-Phenyl-N-methylindolyl)methyl]nitrobenzol

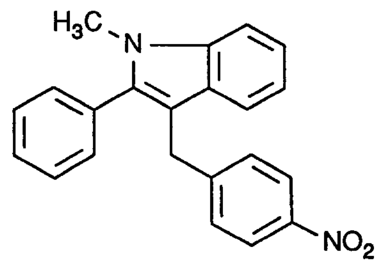

Eine Lösung von 1,53 g (4,7 mmol) p-[3-(2-Phenyl-1H-indolyl)methyl]nitrobenzol in 15 mi DMF wird mit 1,3 g (9,3 mmol) $K_2CO_3$ versetzt und 15 min gerührt. Nach Zutropfen von 0,3 ml (4,7 mmol) Jodmethan wird noch 2 Tage gerührt. Anschließend wird vom Ungelösten abfiltriert, eingeengt und über Kieselgel 60 (Petrolether / Essigester = 10:1) chromatographiert.
Ausbeute: 1,3 g (3,7 mmol) 79% d.Th.
$R_f$ = 0,64 (Petrolether/Essigester = 6:1)

Beispiel V

p-[2-(n-Butyl-1H-benzimidazolyl)methyl]anilin

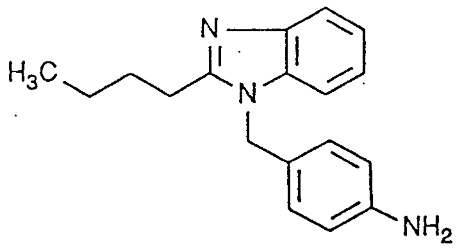

Eine Lösung von 72,8 g des Rohproduktes aus Beispiel II in 500 ml THF wird, verteilt auf 4 Ansätze, mit insgesamt 1l 1N (1 mol) wäßriger $TiCl_3$-Lösung versetzt und 1 h bei 0,1 bar geschüttelt. Anschließend werden 0,8 l $CH_2Cl_2$ zugegeben. Mit halbkonzentriertem Ammoniak wird auf pH 9 gebracht und vom Ungelösten abfiltriert. Der Niederschlag wird erneut mit 0,8 l $CH_2Cl_2$ verrührt und filtriert. Die vereinigten organischen Phasen werden auf 500 ml eingeengt, mit dem gleichen Volumen gesättigter $Na_2SO_4$ getrocknet, filtriert und eingeengt.
Ausbeute: 42 g (0,15 mol) 75% d.Th. über 2 Stufen
$R_f$ = 0,34 ($CH_2Cl_2$ / $CH_3OH$ = 95:5)

Beispiel VI

p-(2-Chinolinylmethylenoxy)-nitrobenzol

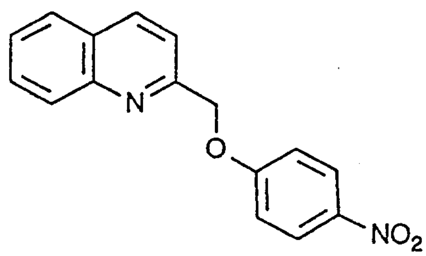

Eine Lösung von 13 g (0,33 mol) NaOH in 400 ml Ethanol wird bei 80 °C unter Rühren mit 44 g (0,32 mol) p-Nitrophenol versetzt und zur Trockne eingedampft. Der Rückstand wird in DMF aufgenommen und zu einer Lösung von 52 g (0,3 mol) 2-Chlormethylchinolin gegeben. Nach 1 h Rühren bei 120 °C wird mit 700 ml $H_2O$ verdünnt, der Niederschlag abfiltriert, mit Wasser gewaschen und in $CH_2Cl_2$ gelöst. Nach Ausschütteln mit Wasser wird die organische Phase getrocknet, eingeengt und das Produkt über Kieselgel 60 mit Dichlormethan / Essigester = 100:1,5 chromatographiert.
Ausbeute: 74 g (0,26 mol) 88% d.Th.
SMP = 147 °C
$R_f$ = 0,44 ($CH_2Cl_2$)

Beispiel VII

p-(2-Chinolinylmethylenoxy)-anilin

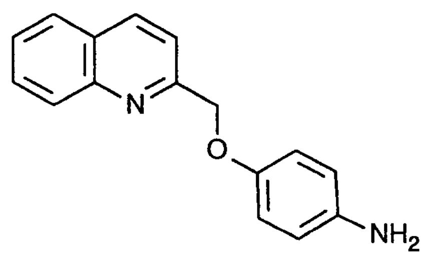

Zu einer Suspension von 79 g (0,28 mol) der Verbindung aus Beispiel VI in 100 ml Methanol und 100 ml THF werden bei 30 °C unter Rühren zunächst 5 g (0,1 mol) $N_2H_4/H_2O$, anschließend in kleinen Portionen Raney-Nickel in Ethanol zugegeben. Nach Ende des Temperaturanstiegs wird 1 h refluxiert. Dann wird filtriert, eingeengt, in $CH_2Cl_2$ aufgenommen, mit Wasser geschüttelt, die organische Phase abgetrennt, getrocknet, eingeengt und das Produkt aus i-Propylether/$CH_2Cl_2$ umkristallisiert.
Ausbeute: 69,2 g (0,276 mol) 98% d.Th.
SMP: 134 °C
$R_f$ = 0,39 ($CH_2Cl_2$/Essigester = 7:3)

Beispiel VIII

p-(2-Chinolinylmethylenoxy)-N-cyclopentylanilin

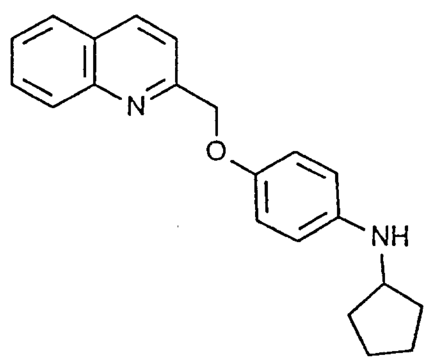

12,5 g (50 mmol) der Verbindung aus Beispiel VII werden in 120 ml THF bei -78 °C unter Argon vorgelegt. Nach Zutropfen von 31,3 ml 1,6 N (50 mmol) n-BuLi in n-Hexan wird 15 min nachgerührt, 7,5 g Cyclopentylbromid zugegeben, 15 min bei -78 °C, 1 h bei -20 °C und 2,5 Tage bei Raumtemperatur gerührt. Nach Verdünnen mit THF wird 3 mal mit NaCl/$H_2O$ geschüttelt, und die vereinigten wäßrigen Phasen werden 1 mal mit THF extrahiert Die vereinigten organischen Phasen werden getrocknet über $Na_2SO_4$, filtriert, eingeengt und das Produkt wird chromatographisch gereinigt (Kieselgel 60, Petrolether/Essigester = 2:1).
Ausbeute: 5,4g (17 mmol) 34% d.Th.
$R_f$ = 0,53 (Petrolether/Essigester = 7:3)

Herstellungsbeispiele

Beispiel 1 und Beispiel 2

N-[p-(2-Chinolinylmethylenoxy)phenyl]-N-cyclopentyl-N'-[S-α-methoxycarbonyl) benzyl)]harnstoff

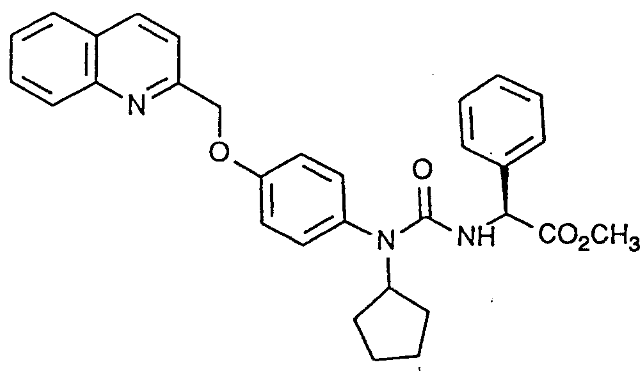

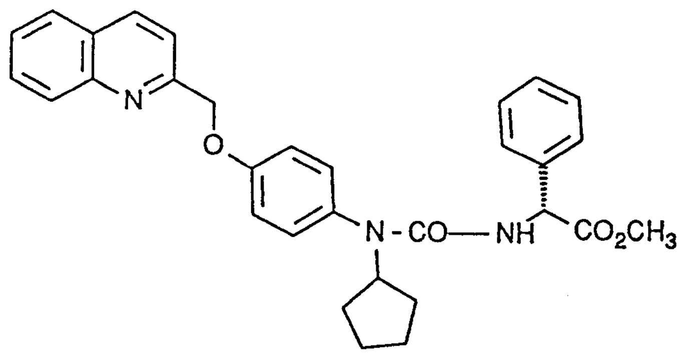

Zu einer Lösung von 2,0 g (6,3 mmol) der Verbindung aus Beispiel VIII in 25 ml THF werden unter Argon bei -78 °C 3,9 ml 1,6 N (6,3 mmol) n-BuLi in n-Hexan unter Rühren zugetropft. Nach 15 min wird über einen -78 °C gekühlten Tropftrichter eine Lösung von 3,4 g (17 mmol) L-(2-Isocyanato)-phenylessigsäuremethylester (hergestellt nach Standardmethoden) zugetropft. 30 min wird bei -40 °C, in den nächsten 30 min bei -15 °C und zuletzt 1,5 h bei Raumtemperatur gerührt. Anschließend wird auf gesättigte $NH_4Cl$-Lösung gegeben, 2 mal mit Essigester extrahiert, vereinigt, getrocknet, filtriert, eingeengt und chromatographiert (Kieselgel 60, Essigester/Petrolether = 1:1).

Ausbeute: 1,8 g (3,5 mmol) 56% d.Th. (Beispiel 1)

$R_f$ = 0,68 (Petrolether/Essigester = 1:1) (Beispiel 1)

$R_f$ = 0,68 (A) (Beispiel 2)

In Analogie zur Vorschrift der Beispiele 1 und 2 werden die in Tabelle 1 aufgeführten Verbindungen hergestellt:

Tabelle 1:

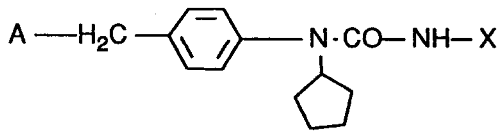

| Bsp,-Nr. | A | X | $R_f$ (LM) |
|---|---|---|---|
| 3 | 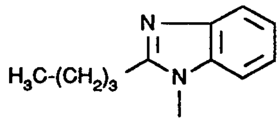 | 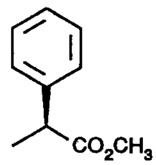 | 0,44 (A) |
| 4 | 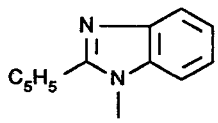 | 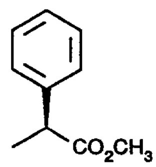 | 0,46 (A) |
| 5 | 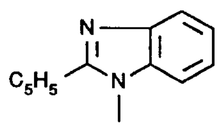 | 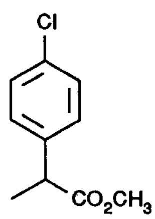 | 0,43 (A) |

Fortsetzung Tabelle 1:

| Bsp.-Nr. | A | X | $R_f$ (LM) |
|---|---|---|---|
| 6 | 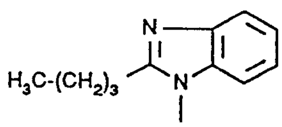 | 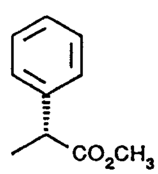 | 0,50 (B) |
| 7 | 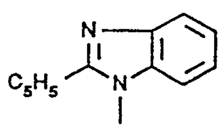 | 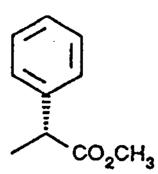 | 0,69 (E) |

Beispiel 8 und Beispiel 9

N-[p-(2-Chinolinylmethylenoxy)-phenyl]-N-cyclopentyl-N'-[S-(α-hydroxymethyl)benzyl]harnstoff

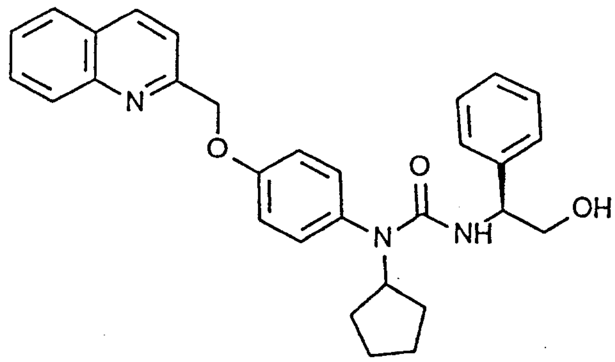

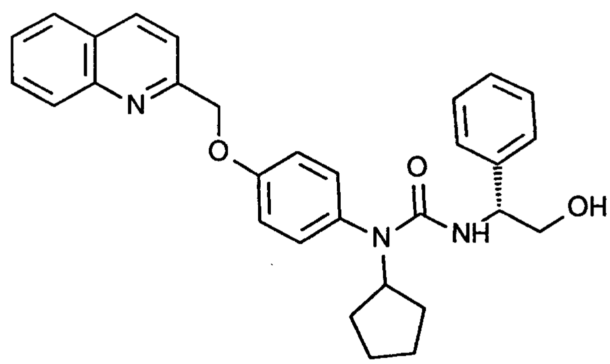

Zu einer Lösung von 2,6 ml 1 N (2,6 mmol in THF) $LiAlH_4$ in 4 ml THF wird unter Argon bei Raumtemperatur eine Lösung von 0,5 g (0,98 mmol) der Verbindung aus Beispiel 1 in 6 ml THF gegeben und 1 h gerührt. Anschließend wird langsam in 60 ml Eiswasser gegossen, mit Essigsäure auf pH 5 eingestellt, dreimal mit Essigester extrahiert, und die vereinigten organischen Phasen werden über $Na_2SO_4$ getrocknet, filtriert und eingeengt. Das Produkt wird über Kieselgel 60 (Toluol/Essigester = 1:2) gereinigt.
Ausbeute: 120 mg (25 mmol) 25% d.Th. (Beispiel 8)
$R_f$ = 0,49 (Toluol/Essigester = 1:3) (Beispiel 8)
$R_f$ = 0,49 (C) (Beispiel 9)
In Analogie zur Vorschrift der Beispiele 8 und 9 werden die in Tabelle 2 aufgeführten Verbindungen hergestellt:

Tabelle 2:

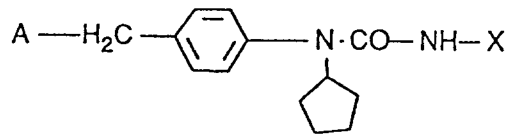

| Bsp.-Nr. | A | X | $R_f$ (LM) |
|---|---|---|---|
| 10 | 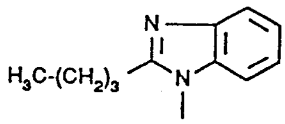 | 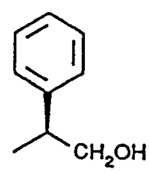 | 0,30 (C) |
| 11 | 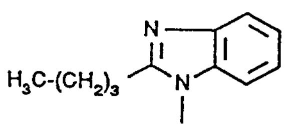 | 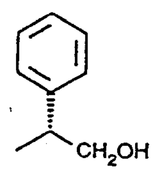 | 0,30 (C) |
| 12 | 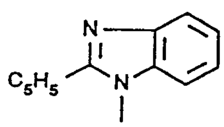 | 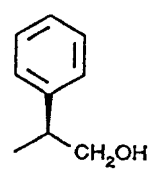 | 0,38 (C) |

Fortetzung Tabelle 2:

| Bsp.-Nr. | A | X | $R_f$ (LM) |
|---|---|---|---|
| 13 | 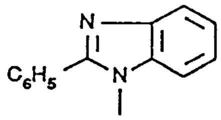 | 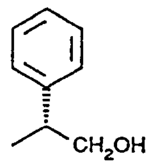 | 0,14 (F) |
| 14 | 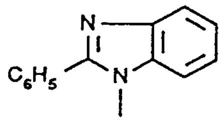 | 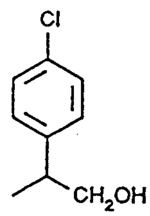 | 0,33 (C) |
| 15 | 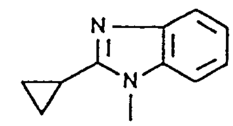 | 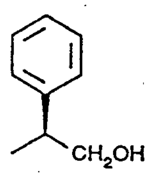 | 0,23 (D) |
| 16 | 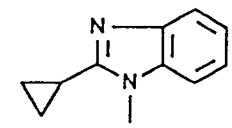 | 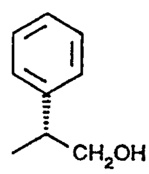 | 0,23 (D) |
| 17 | 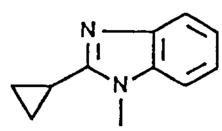 | 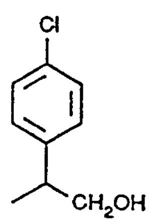 | 0,17 (D) |

Fortetzung Tabelle 2

| Bsp.-Nr. | A | X | $R_f$ (LM) |
|---|---|---|---|
| 18 | 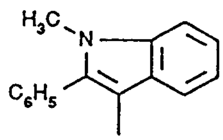 | 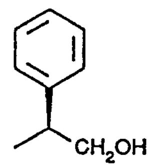 | 0,33 (A) |
| 19 | 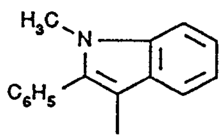 | 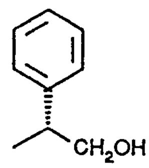 | 0,33 (A) |
| 20 | H | 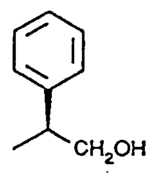 | 0,26 (F) |

Beispiel 21

N-[p-(2-Chinolinylmethylenoxy)phenyl]-N-cyclopentyl-N'-[S-(2-phenyl)-acetamido]harnstoff

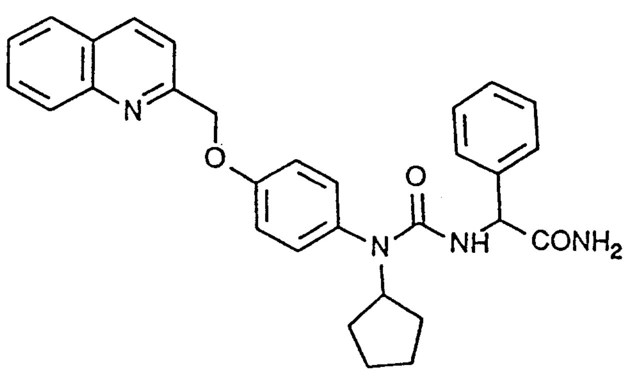

Zu einer Lösung von 0,5 g (0,98 mmol) der Verbindung aus Beispiel 1 in 6 ml Methanol wird eine gesättigte Lösung von Ammoniak in 25 ml Methanol gegeben und 2 Tage bei Raumtemperatur gerührt. Nach Einengen wird das Produkt über Kieselgel 60 (Essigester/Petrolether = 7:3) gereinigt.

Ausbeute: 0,3 g (0,61 mmol) 62% d.Th.

$R_f$ = 0,35 (Petrolether/Essigester = 3:7)

In Analogie zur Vorschrift des Beispiels 21 werden die in Tabelle 3 aufgeführten Verbindungen hergestellt:

Tabelle 3:

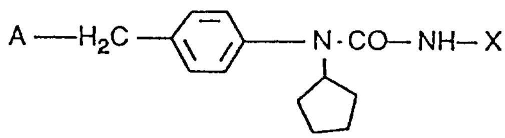

| Bsp.-Nr. | A | X | $R_f$ (LM) |
|---|---|---|---|
| 22 | 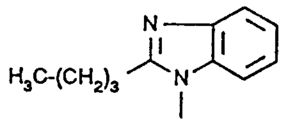 | 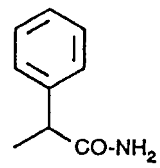 | 0,58 (D) |
| 23 | 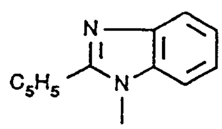 | 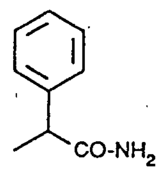 | 0,41 (D) |
| 24 | 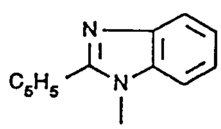 | 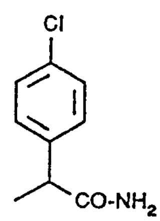 | 0,38 (C) |

Fortsetzung Tabelle 3:

| Bsp.-Nr. | A | X | $R_f$ (LM) |
|---|---|---|---|
| 25 | 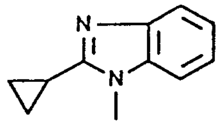 | 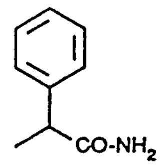 | 0,26 (D) |
| 26 | 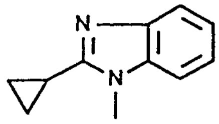 | 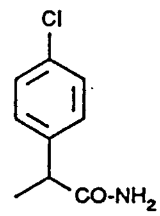 | 0,18 (D) |
| 27 | 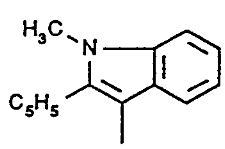 | 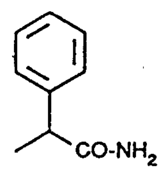 | 0,63 (G) |
| 28 | H | 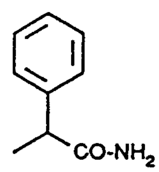 | 0,17 (F) |

**Patentansprüche**

**1.** Substituierte Arylharnstoffe der allgemeinen Formel (I)

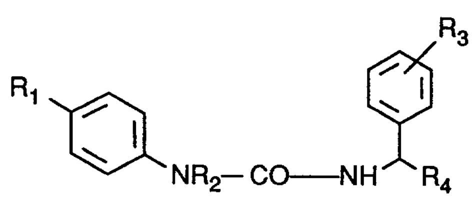

(I)

in welcher

$R^1$ für einen Rest der Formel $-CH_2$-A oder $-O-CH_2$-D steht, worin

A und D gleich oder verschieden sind und Wasserstoff oder einen 5- bis 7-gliedrigen, gesättigten oder ungesättigten Heterocyclus mit bis zu 3 Stickstoffatomen bedeuten, an den gegebenenfalls ein Phenyl- oder Pyridylring ankondensiert ist, und wobei die Bindung an die Methylengruppe im Fall eines N-haltigen Ringes auch über die Stickstofffunktion erfolgen kann, und wobei gegebenenfalls die Cyclen bis zu 3-fach gleich oder verschieden durch geradkettiges oder verzweigtes Alkyl, Alkoxy oder Alkoxycarbonyl mit jeweils bis zu 8 Kohlenstoffatomen, Cycloalkyl mit 3 bis 6 Kohlenstoffatomen, Phenyl Hydroxy, Carboxy oder Halogen substituiert sind,

$R^2$ für Cycloalkyl mit 3 bis 7 Kohlenstoffatomen oder für geradkettiges oder verzweigtes Alkyl mit bis zu 8 Kohlenstoffatomen steht,

$R^3$ für Wasserstoff oder Halogen steht,

und

$R^4$ für Carboxy oder geradkettiges oder verzweigtes Alkoxycarbonyl mit bis zu 8 Kohlenstoffatomen steht, oder
für geradkettiges oder verzweigtes Alkyl mit bis zu 6 Kohlenstoffatomen steht, das durch Hydroxy substituiert ist, oder
für eine Gruppe der Formel $-CO-NR^5R^6$ steht,
worin

$R^5$ und $R^6$ gleich oder verschieden sind und Wasserstoff oder geradkettiges oder verzweigtes Alkyl mit bis zu 6 Kohlenstoffatomen bedeuten,

und deren Salze.

**2.** Substituierte Arylharnstoffe nach Anspruch 1
worin

$R^1$ für einen Rest der Formel $-CH_2-A$ oder $-O-CH_2-D$ steht,
worin

A und D gleich oder verschieden sind und Wasserstoff, Benzimidazolyl, 3H-Imidazopyridyl, 3-Indolyl oder 2-Chinolinyl bedeuten, die sowohl im Phenyl als auch im heterocyclischen Rest gegebenenfalls bis zu 2-fach gleich oder verschieden durch geradkettiges oder verzweigtes Alkyl, Alkoxy oder Alkoxycarbonyl mit jeweils bis zu 6 Kohlenstoffatomen, Cyclopropyl, Cyclopentyl, Cyclohexyl, Hydroxy, Phenyl, Carboxy, Fluor, Chlor oder Brom substituiert sind,

$R^2$ für Cyclopropyl, Cyclopentyl, Cyclohexyl, Cycloheptyl, oder
für geradkettiges oder verzweigtes Alkyl mit bis zu 6 Kohlenstoffatomen steht,

$R^3$ für Wasserstoff, Fluor, Chlor oder Brom steht,

und

$R^4$ für Carboxy oder geradkettiges oder verzweigtes Alkoxycarbonyl mit bis zu 6 Kohlenstoffatomen oder
für geradkettiges oder verzweigtes Alkyl mit bis zu 4 Kohlenstoffatomen steht, das durch Hydroxy substituiert ist, oder
für eine Gruppe der Formel $-CO-NR^5R^6$ steht,
worin

$R^5$ und $R^6$ gleich oder verschieden sind und Wasserstoff oder geradkettiges oder verzweigtes Alkyl mit bis zu 4 Kohlenstoffatomen bedeuten

und deren Salze.

**3.** Substituierte Arylharnstoffe nach Anspruch 1
worin

$R^1$ für einen Rest der Formel $-CH_2-A$ oder $-O-CH_2-D$ steht,
worin

A und D gleich oder verschieden sind und Wasserstoff, Benzimidazolyl, 3H-Imidazopyridyl, 3-Indolyl oder 2-Chinolinyl bedeuten, die sowohl im Phenyl als auch im heterocyclischen Rest gegebenenfalls bis zu 2-fach gleich oder verschieden durch geradkettiges oder verzweigtes Alkyl, Alkoxy oder Alkoxycarbonyl mit jeweils bis zu 5 Kohlenstoffatomen, Cyclopropyl, Cyclopentyl, Cyclohexyl, Hydroxy, Phenyl oder Carboxy substituiert sind,

$R^2$ für Cyclopropyl, Cyclopentyl, Cyclohexyl, Cycloheptyl, oder
für geradkettiges oder verzweigtes Alkyl mit bis zu 4 Kohlenstoffatomen steht,

$R^3$ für Wasserstoff, Fluor, Chlor oder Brom steht,

und

$R^4$ für Carboxy oder geradkettiges oder verzweigtes Alkoxycarbonyl mit bis zu 3 Kohlenstoffatomen oder
für geradkettiges oder verzweigtes Alkyl mit bis zu 3 Kohlenstoffatomen steht, das durch Hydroxy substituiert ist, oder
für eine Gruppe der Formel $-CO-NR^5R^6$ steht,
worin

$R^5$ und $R^6$ gleich oder verschieden sind und Wasserstoff oder geradkettiges oder verzweigtes

Alkyl mit bis zu 3 Kohlenstoffatomen bedeuten

und deren Salze.

**4.** Substituierte Arylharnstoffe nach Anspruch 1
worin
$R^1$ für Wasserstoff oder für den Rest der Formel

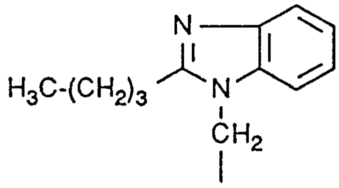

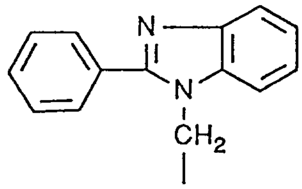

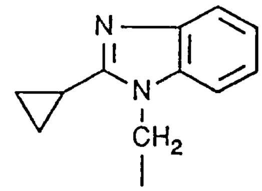

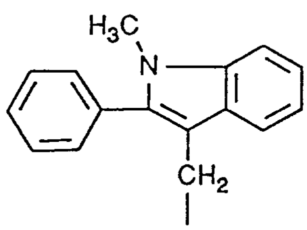

oder

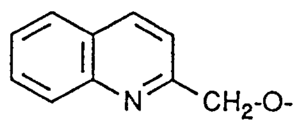

steht.

**5.** Substituierte Arylharnstoffe nach Ansprüchen 1 bis 4 zur therapeutischen Anwendung.

**6.** Verfahren zur Herstellung von substituierten Arylharnstoffen nach Ansprüchen 1 bis 4, dadurch gekennzeichnet, daß man Verbindungen der allgemeinen Formel (II)

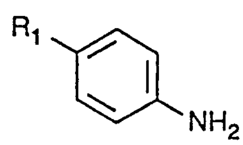

(II)

in welcher
$R^1$ die oben angegebene Bedeutung hat,
zunächst durch Umsetzung mit Verbindungen der allgemeinen Formel (III)

$R^2$-L (III)

in welcher
$R^2$ die oben angegebene Bedeutung hat
und
L für Halogen, vorzugsweise für Brom steht,
in inerten Lösemitteln, in Anwesenheit einer Base und unter Schutzgasatmosphäre in die Verbindungen der allgemeinen Formel (IV)

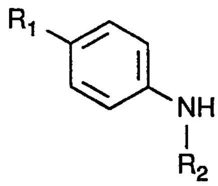

(IV)

in welcher
$R^1$ und $R^2$ die oben angegebene Bedeutung haben,
überführt,
und in einem zweiten Schritt mit Verbindungen der allgemeinen Formel (V)

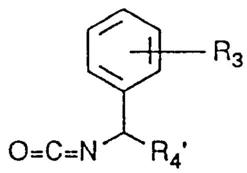

(V)

in welcher
$R^3$ die oben angegebene Bedeutung hat
und
$R^{4'}$ für geradkettiges oder verzweigtes Alkoxycarbonyl mit bis zu 8 Kohlenstoffatomen steht,
in inerten Lösemitteln, gegebenenfalls in Anwesenheit einer Base und unter Schutzgasatmosphäre umsetzt,
und im Fall der Säuren $R^4$ = $CO_2H$, die Ester verseift,
im Fall der Alkylhydroxyverbindungen die Ester nach üblichen Bedingungen reduziert,
und im Fall der Amide die Ester oder Säuren entweder mit Ammoniak ($R^4$ = $CO\text{-}NH_2$) oder mit Aminen, gegebenenfalls nach vorgeschalteter Aktivierung der Carbonsäurefunktion, umsetzt.

**7.** Arzneimittel enthaltend mindestens einen substituierten Arylharnstoff nach Ansprüchen 1 bis 4.

**8.** Arzneimittel nach Anspruch 7 zum Inhibieren der Proliferation glatter Muskelzellen.

**9.** Arzneimittel nach Anspruch 7 zur Behandlung von Arteriosklerose und Restenose.

**10.** Verwendung von substituierten Arylharnstoffen nach Ansprüchen 1 bis 4 zur Herstellung von Arzneimitteln.

Europäisches Patentamt

# EUROPÄISCHER RECHERCHENBERICHT

Nummer der Anmeldung
EP 95 10 0310

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | WO-A-92 10468 (WELLCOME FOUND ;UNIV SOUTH CAROLINA (US)) 25.Juni 1992<br>* das ganze Dokument *<br>--- | 1-10 | C07D235/14<br>A61K31/17<br>C07D215/12<br>C07D209/10 |
| X | WO-A-93 24458 (PFIZER ;HAMANAKA ERNEST S (US)) 9.Dezember 1993<br>* das ganze Dokument *<br>--- | 1-10 | |
| Y | US-A-4 623 662 (DE VRIES VERN G) 18.November 1986<br>* das ganze Dokument *<br>--- | 1-10 | |
| Y | EP-A-0 418 071 (PFIZER) 20.März 1991<br>* das ganze Dokument *<br>--- | 1-10 | |
| Y | EP-A-0 384 320 (WARNER LAMBERT CO) 29.August 1990<br>* das ganze Dokument *<br>--- | 1-10 | |
| Y | EP-A-0 335 375 (WARNER LAMBERT CO) 4.Oktober 1989<br>* das ganze Dokument *<br>--- | 1-10 | **RECHERCHIERTE SACHGEBIETE (Int.Cl.6)** |
| Y | EP-A-0 293 880 (WARNER LAMBERT CO) 7.Dezember 1988<br>* das ganze Dokument *<br>--- | 1-10 | C07D<br>A61K |
| Y | EP-A-0 506 532 (LIPHA) 30.September 1992<br>* das ganze Dokument *<br>--- | 1-10 | |
| Y | EP-A-0 447 116 (YAMANOUCHI PHARMA CO LTD) 18.September 1991<br>* das ganze Dokument *<br>--- | 1-10 | |
| Y | EP-A-0 399 422 (TAKEDA CHEMICAL INDUSTRIES LTD) 28.November 1990<br>* das ganze Dokument *<br>----- | 1-10 | |

Der vorliegende Recherchenbericht wurde für alle Patentansprüche erstellt

| Recherchenort | Abschlußdatum der Recherche | Prüfer |
|---|---|---|
| MÜNCHEN | 2.Juni 1995 | Stellmach, J |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument